# EUROPEAN PATENT SPECIFICATION

(11) **EP 3 076 862 B1**
(45) Date of publication and mention of the grant of the patent: **12.06.2019**
(21) Application number: 14816122.7
(22) Date of filing: 05.12.2014
(51) Int. Cl.: A61B 5/0205, A61B 5/083

(54) **INTELLIGENT MEDICAL MONITORING OF A PATIENT**
INTELLIGENTE MEDIZINISCHE PATIENTENÜBERWACHUNG
SURVEILLANCE MÉDICALE INTELLIGENTE D'UN PATIENT

(30) Priority: 05.12.2013 DK 201370748
(43) Date of publication of application: 12.10.2016
(73) Proprietor: Mermaid Care A/S, 9400 Nørresundby (DK)
(72) Inventor: REES, Stephen Edward, 9260 Gistrup (DK); KARBING, Dan Stieper, 9000 Aalborg (DK)
(74) Representative: Høiberg P/S
(86) International application number: PCT/DK2014/050414
(87) International publication number: WO 2015/081965

(56) References cited:
- EP-A1- 2 098 163
- WO-A1-02/02006
- WO-A2-2009/063446
- US-A1- 2010 073 170
- STEPHEN EDWARD REES: "THE AUTOMATIC LUNG PARAMETER ESTIMATOR (ALPE) SYSTEM: NON-INVASIVE ESTIMATION OF PULMONARY GAS EXCHANGE PARAMETERS IN 10-15 MINUTES", JOURNAL OF CLINICAL MONITORING AND COMPUTING, vol. 17, 1 January 2002 (2002-01-01), pages 43-52, XP055003350,

## Description

### FIELD OF THE INVENTION

The present invention relates to a computer system and a corresponding method for medical monitoring of a patient, wherein mathematical models are used to provide suggestions for the causes of changes in measured values from the patient or even identify worsening of the patient in the presence or absence of changes in the measured values from the patient.

### BACKGROUND OF THE INVENTION

Patients with severe acute illness, often requiring mechanical support for ventilation, usually reside in the intensive care unit (ICU) of the hospital. Here they are intensively monitored using a range of machines which measure physiological variables related to vital functions. These include measurement of the oxygenation and acid-base status of the blood, circulation, metabolism, respiratory function etc.

These measurements are interpreted by the clinician who then assesses the state of the patient and determines whether a change in therapeutic intervention is necessary. The measurement instruments can, in some instances, support the clinician in this interpretation through the use of alarms. For example, reduction in arterial oxygenation beyond a certain level e.g. 90% which is usually monitored by a pulse oximeter finger clip, can cause an alarm. The same is true of a reduction in blood pressure in the measurement of circulation, or an increase in inspiratory pressure in the measurement of pulmonary function and numerous similar alarms.

Common amongst them is that these alarms are based upon the variation in a single measured variable characterizing a single physiological system and often from the single device on which the measurement was taken.

A further type of alarm present in the ICU is that due to sensor detachment or equipment failure. Patients can often be restless and these alarms are therefore necessary and common in, for example, the situation where a pulse oximeter detaches from the patients finger. However, the detachment itself of such a sensor can be difficult to distinguishing from a change in physiological state of the patient.

US patent application 2007/0000494 (invented by Michael J. Banner et al., assigned to University of Florida Research Foundation) discloses a ventilator monitoring system and a method of using same. The invention described and shown in the specification and drawings include a system and method for monitoring the ventilation support provided by a ventilator that is supplying a breathing gas to a patient via a breathing circuit that is in fluid communication with the lungs of the patient. To solve the problem of appropriate adjustments of the ventilation support and advice to clinician in that respect, a neural network adapted for learning from earlier patient data is further described. After so-called training of the neural network implemented on a processor, it may be applied for recommendations of ventilation support settings to a clinician being faced with the task of adjusting ventilation settings. However, the described system also fails to provide more intelligent information behind any deviations or abnormalities observed during mechanical ventilation of a patient.

Rees et al.: "The automatic lung parameter estimator (ALPE) system: Non-invasive estimation of pulmonary gas exchange parameters in 10-15 minutes" shows a patient monitoring system that uses a model to define the functioning of pulmonary gas exchange.

Hence, an improved monitoring method would be advantageous, and in particular a more efficient and/or reliable method would be advantageous.

### OBJECT OF THE INVENTION

The invention is defined in the appended claims, the description being for illustrative purposes only.

It is a further object of the present invention to provide an alternative to the prior art. In particular, it may be seen as an object of the present invention to provide a computer system and a corresponding method that solves the above mentioned problems of the prior art with monitoring of patients, particular patients in intensive care units (ICUs).

### SUMMARY OF THE INVENTION

The prior art alarm strategies, based upon single measurements or sensor detachment, are intended to aid in monitoring the patient. However, these strategies may have drawbacks.

Firstly, alarms based on single measurement variables are a poor help in aiding the clinician in understanding the deeper cause of the problem. For example, a decrease in oxygenation of arterial blood can be due to numerous causes. The patient may have become active or developed a fever, in which case metabolic demand may have increased. The patient's lung function may have deteriorated such that oxygen transports less freely from the lungs to blood, or it may just be the case that the clinician has inadvertently reduced inspired oxygen to a level where arterial values are compromised. Measurements and alarms indicating reduced arterial oxygenation are therefore useful but are not the complete picture.

Secondly, current monitoring and alarm strategies do not direct the clinician as to the requirement for further measurement. For example, an increase in carbon dioxide levels in expiratory gas, can indicate increased metabolic production of CO2, a reduced respiration, or a change in the acid-base status of the blood.

Furthermore, clinicians may get stressed by the vast number of settings, physiological parameters, screens, and the relationship between these values and their impact on therapeutic decisions in relation to conflicting goals. Stress and lack of overview can lead to errors in an ICU [1].

Thus, the above described object and several other objects are intended to be obtained in a first aspect of the invention by providing a computer system for medical monitoring of a patient using one or more physiological models implemented on the computer system, the system being arranged for:
- providing first data indicative of characteristics in the inspired gas of the patient to the computer system ,
- providing second data indicative of characteristics in the respiratory feedback in expired gas of the patient to the computer system, and
- providing third data indicative of the acid/base status and oxygenation level in the blood of the patient to the computer system,
the computer system being arranged for:
i. performing a mathematical physiological modelling of the patient using said first, second and third data as a function of time, wherein the one or more physiological models being capable of modelling the patient over time by outputting a plurality of corresponding physiological parameters derivable from said first, said second and said third data describing the functioning of at least two organ systems and/or physiological systems simultaneous,
ii. performing a monitoring process wherein
   - if one, or more, of the physiological parameters have a deviation compared to a related threshold level, the computer system outputs:
      - an indication of the one or more organ systems, and/or one or more physiological systems causing said deviation;
      - and optionally, an alarm signal ;
      and/or
   - if one or more of the data values have a deviation compared to a related threshold level, the computer system outputs:
      - an indication of one or more organ systems, and/or one or more physiological systems and/or one or more non-physiological systems causing said deviation;
      - optionally, an alarm signal.

Thus, the computer system of the invention provides an indication for the underlying cause for a change in a measured value from a patient. Furthermore, the computer system is also able to provide an indication of underlying physiological changes of the patient in a case where the data values are still inside threshold ranges. This may allow the clinician to take action even before measured values have changed.

In the context of the present invention, the physiological parameters being the result of the modelling may be obtained by fitting the models to the said data. The fitting may be performed numerically and/or analytically. The fitting may be performed continuously, regularly, or at user-defined intervals, possibly in a combination of these time regimes of fitting.

It is to be understood that the indication of the one or more organ systems, and/or one or more physiological systems and/or one or more non-physiological systems causing said deviation may be one possible cause, or a plurality of possible causes for the deviation. It is further to be understood that computer system may be arranged for further weighting a probability of a certain cause before outputting the possible cause to a user, e.g. a clinician. In some cases, the actual probability for a cause is unknown (but above zero), and the possible causes may then all be outputted and listed to a user. Thus, if below a certain probability the cause may be not be outputted to a user. Alternatively or additionally, the cause may be prioritized according their associated probabilities.

It may be mentioned that - in the context of the present invention - the said indications are intended for assisting or guiding e.g. clinician in making decisions of a therapeutic and/or a diagnostic character. Thus, the present invention is not designed to perform an actual diagnosis, merely providing intelligent information, i.e. indications that may assist the clinician in performing the subsequent step of making the intellectual exercise of providing a diagnosis of the patient state, the diagnosis may then be followed be an action of therapeutic character, if needed.

In the context of the present invention, the patient may be a mammal, such as a human, a monkey, a horse, a rodent, etc.

As mentioned above, a change in a data signal to a level above or below a predetermined threshold level may have different causes, making it difficult for the clinician to make the optimal choice in relation to treatment. Table 1 below list a number of non-exclusive examples of both physiological and non-physiological causes for a change in a data signal.

**Table 1:**

| | Possible causes of changes in data | |
|---|---|---|
| **Data signal** | **Physiologic** | **Non-physiologic** |
| | | |
| SpO2 low | Lung abnormality, Metabolism increase | sensor malfunction, Inspired oxygen low |
| FetCO2 high | Lung abnormality, Metabolism increase, reduced respiratory drive, respiratory muscle fatique | sensor malfunction, Reduced ventilation volume |
| Mean arterial blood pressure low | Low circulatory volume, high pulmonary pressures, heart function abnormalities | sensor malfunction |
| Positive inspiratory pressure high | Poor lung mechanics | sensor malfunction, Increase in mechanical ventilation. |
| Blood Base excess change | Kidney bicarbonate reabsorption, Anaerobic metabolism, ketoacidosis | Blood gas measurement error. |

Thus, in an embodiment of the invention the observed deviation is a SpO2 level below a threshold level and
- the indication of a physiological cause is selected from the group consisting of lung abnormality and metabolism increase; and/or
- the indication of a non-physiological cause is selected from the group consisting of a sensor malfunction and a low level of inspired oxygen.

In another embodiment of the invention the observed deviation is a FetCO2 level above a threshold level and
- the indication of a physiological cause is selected from the group consisting of lung abnormality, metabolism increase, reduced respiratory drive and respiratory muscle fatigue; and/or
- the indication of a non-physiological cause is selected from the group consisting of a sensor malfunction and reduced ventilation volume.

In yet another embodiment of the invention the observed deviation is a mean arterial blood pressure below a threshold level and
- the indication of a physiological cause is selected from the group consisting of low circulatory volume, high pulmonary pressures, and heart function abnormalities; and/or
- the indication of a non-physiological cause is a sensor malfunction.

In yet an embodiment of the invention the observed deviation is a positive inspiratory pressure above a threshold level
- and the indication of a physiological cause is poor lung mechanics; and/or
- the indication of a non-physiological cause is selected from the group consisting of a sensor malfunction and a low level of inspired oxygen and increase in mechanical ventilation.

The indication may be based on different values. Thus, in an embodiment the indication of one or more organ systems, one or more physiological systems and/or non-physiological systems causing said deviation is based on
▪ current values of other physiological parameters and/or current data values; and/or
▪ previous values of the deviating physiological parameter and/or previous values of the data values.

The computer system of the invention may employ further data input. Thus, in yet an embodiment the computer system is further arranged for
- providing fourth data indicative of characteristics of the blood circulation, such as cardiac output; and/or
- providing fifth data indicative of characteristics of heart function, such as blood pressure; and/or
- providing sixth data indicative of characteristics of kidney function, such as renal perfusion or bicarbonate reabsorbtion.

The term "organ systems" covers different organs of the patient. In an embodiment the one or more organ systems are chosen from the group consisting of lungs, blood, heart, kidney and tissues.

The term "physiological systems" covers different physiological systems of the patient. Thus, in a further embodiment the one or more physiological systems are chosen from the non-exhaustive group consisting of metabolism, circulation, respiration and renal function and used in a modelling of the patient, i.e. physiological modelling over time It is to be understood that the modelling over time may particularly include the possibility of predicting or estimating one, or more, parameters at a later time.

### Physiological model:

In the present context the term "physiological model" should be understood as the broadest sense of applied physiological models, the term physiological model here covering any means for linking clinically measurable values mathematically and for the individual patient, that is, that any parameters that needs tuning for the model to describe the individual patient's physiology should be possible to estimate from clinical measurements. In its simplest form such a physiological model can be a linear model of the equation y = a^{∗}x with a being the parameter of the model and y and x being clinical measureable values such as the link between pressure (x) and volume (y) with lung compliance (a) linking the two (Volume = Pressure^{∗}Compliance). The more complex end of the range include a model encompassing all chemical reaction equations of the buffer systems of human blood with parameters such as rate constants which may be assumed equal for all individuals but with for example Base Excess that should be tuned to the individual patient [2]. The invention may require physiological models of lung mechanics, gas exchange, blood acid-base status, respiratory drive, heart function, kidney function, and/or blood circulation in general.

### Lung Mechanics

A physiological model of lung mechanics links flows, volumes and or pressures which may be measured at the mouth, in the oesophagus, at the ventilator, in the ventilator tube, in the airways etc. More complex physiological models may describe the resistances of the respiratory system and the individual elastic characteristics of the alveoli, the lung tissue, the diaphragm, the surfactant, the chest wall, the airways, etc. In control modes, identification of the overall characteristics of the respiratory system such as compliance is straightforward and is routinely measured by the ventilator. In support mode, the volumes inspired and expired by the patient not only depend on lung mechanics and ventilator settings, but also patient respiratory work. Simple models of lung mechanics in support ventilation may therefore describe an effective compliance, which is the combined pressure volume relationship of ventilator and patient work.

### Gas exchange

A physiological model of gas exchange links measurements of contents of inspired and expired gases to measured contents of these gases in blood (arterial, venous, mixed venous or capillary blood). Often this is mathematically implemented as a set of equations describing the exchange of one, or more, respiratory gases (oxygen, carbon dioxide, nitrogen) or inert gases added to the inspired gas (such as xenon, krypton, nitric oxide, methane etc.) across the alveolar membrane to capillary blood in one or more compartments and the mixing of the blood leaving these compartments with shunted venous blood to constitute the arterial blood, the latter which is often sampled and analysed in the clinical settings. Venous or mixed venous blood may also be sampled in the clinical setting and can also be described by a model by including equations with patient metabolism and blood flow in the model. Estimation of model parameters for gas exchange models, in particular for models with one or more of the aforementioned compartments, requires either invasive measurements such as a mixed venous blood sample from the pulmonary artery or that the gas contents of inspired gas is modified in one or more steps with the patient response to these changes measured continuously and/or at steady state this making it possible to separate the contributions of one or more mechanisms for gas exchange problems these in the model represented by parameters such as intrapulmonary shunt, ventilation/perfusion ratio etc. Gas exchange models often encompass a model of blood acid base status and the binding and transport of respiratory gases in blood.

### Blood acid-base and oxygen and carbon dioxide transport

A model of blood acid-base status and/or oxygen and/or carbon dioxide transport describes either empirically or by reaction equations some or all of the chemical reactions occurring in blood for chemical buffering and binding of gases to haemoglobin. The models may be in so-called steady state describing the reaction equations at equilibrium or dynamical describing the reactions over time. As such, the models link values of blood acid base that can be measured routinely at the bedside (such as oxygen and carbon dioxide pressures and pH) to values not easily obtained (such as base excess, concentrations of buffers, concentrations of respiratory gases etc.).

### Physiological parameters:

A physiological parameter should in this context be understood as a value describing the characteristics of a physiological system and can be assumed constant for changes in some clinical variables and/or ventilator settings. For example, pulmonary shunt describes the fraction of blood flow to the lungs not reaching ventilated alveoli representing the most important gas exchange problem in mechanically ventilated patients. Pulmonary shunt is known to remain constant for variation in a number of clinical variables and ventilator settings such as respiratory frequency and tidal volume.

Below are listed, in an illustrative, non-limiting and non-exhaustively manner, some physiological parameters suitable for application in the present invention:
*Lung mechanics parameters* include lung compliance, respiratory resistances, respiratory system elastance, effective shunt, compliances and resistances of individual components of the respiratory system (alveoli, airways, chest wall, diaphragm, surfactant).
*Gas exchange parameters* include but is not limited to pulmonary shunt, venous admixture, effective shunt, Ventilation/perfusion (V/Q) ratio, degree of low V/Q, degree of high V/Q, ΔPO2, ΔPCO2, Arterial-end tidal O2 difference, Arterial-end tidal CO2 difference, anatomical dead space, alveolar dead space, physiological dead space, end-expiratory lung volume, functional residual capacity etc.
*Blood acid-base parameters* include but is not limited to base excess (BE), strong iron difference (SID), haemoglobin concentration, blood volume, rate constants for chemical reactions etc. This includes examples of third data (D3) according to the present invention.

The computer system of the invention may also comprise certain simulations. Thus, in another embodiment the computer system is arranged for, optionally when no physiological parameters have a deviation compared to a related physiological threshold: - performing a simulation of the patient state by keeping constant one or more, possible every, physiological parameters from the modelling and simulating the time development of the patient state. In yet an embodiment said simulation comprises a tuning process of one, or more, model parameters at regularly intervals, possible continuously at least with respect to the available computational resources or frequency of data measurements. In yet a further embodiment said simulation of the patient state over time is applied for comparing one, or more, physiological parameters and/or simulated physiological values corresponding to obtainable data with actual data to assess if the said simulation can adequately describe the patient state now or at a later time.

Besides providing an indication of an underlying cause for the change in measured values or physiological parameters, the computer system may also allow for providing an indication of further tests from which values may be used in the same models or new models to improve the suggested indications. Thus, in an embodiment the computer system being arranged for indicating one or more further patient measurements which can be performed for obtaining further data values which may improve the modelling, the simulation and/or the provided indication. Such further test may be tests which are usually not constantly measured/obtained during a standard monitoring. Thus, both the type of measurements for additional data and/or timing/frequency of such data may be suggested by the computer system. In yet an embodiment the further tests are selected from the non-exhaustive group consisting of arterial or venous blood gas, blood electrolyte values, blood glucose or ketone concentration, cardiac output measurement or other invasive measurements describing blood, circulation, metabolism, respiration or renal function.

Situations may arise where the computer system or the method (for some reason) is not able to describe the patient state. Thus, in an embodiment, if said simulation cannot describe the patient state now or at later time, the computer system being arranged for indicating one or more possible causes therefore. Possible causes could be errors in the previously measured values such that for example measurement of arterial oxygenation is not consistent with measurement of inspired oxygenation or metabolic demand. In a situation such as this one of these measurements may be in error.

As previously mentioned the method or the computer system may be able to give an indication in a change in a physiological parameter without any significant change has been measured in the data values. Thus, in an embodiment no data values lies outside a threshold range, while a change in one or more physiological parameters (organ systems, and/or one or more physiological systems) are indicated. This may be specifically advantageous since this provides the clinician with a warning of a specific failure which would not otherwise be indicated. Thus, in a similar embodiment no data values deviates significantly from previous provided data values from said patient. Thus, the computer system may be working even
in the absence of changes in data values.

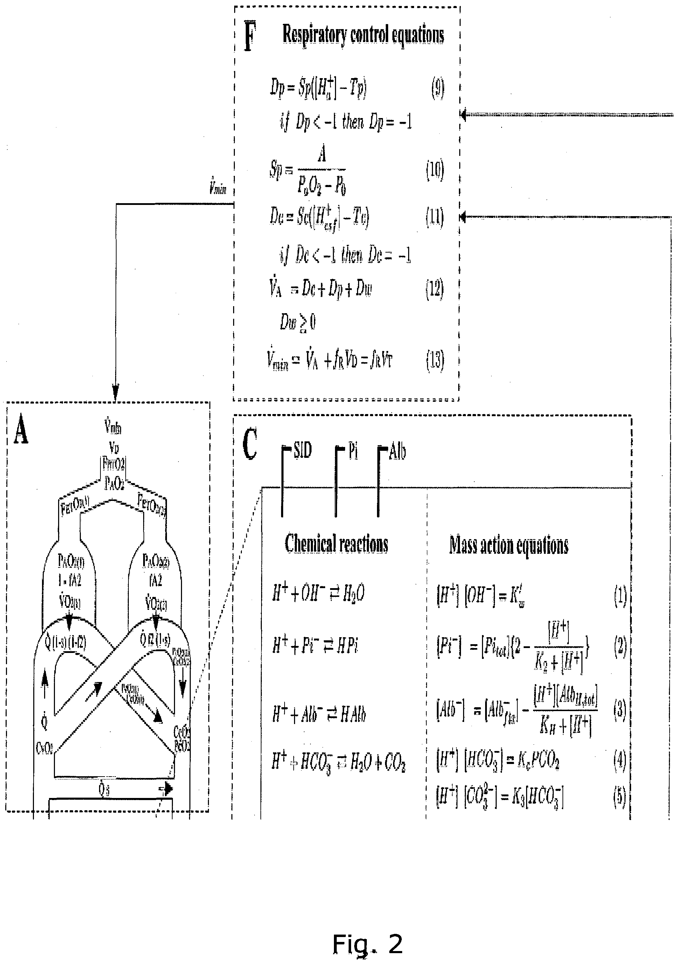

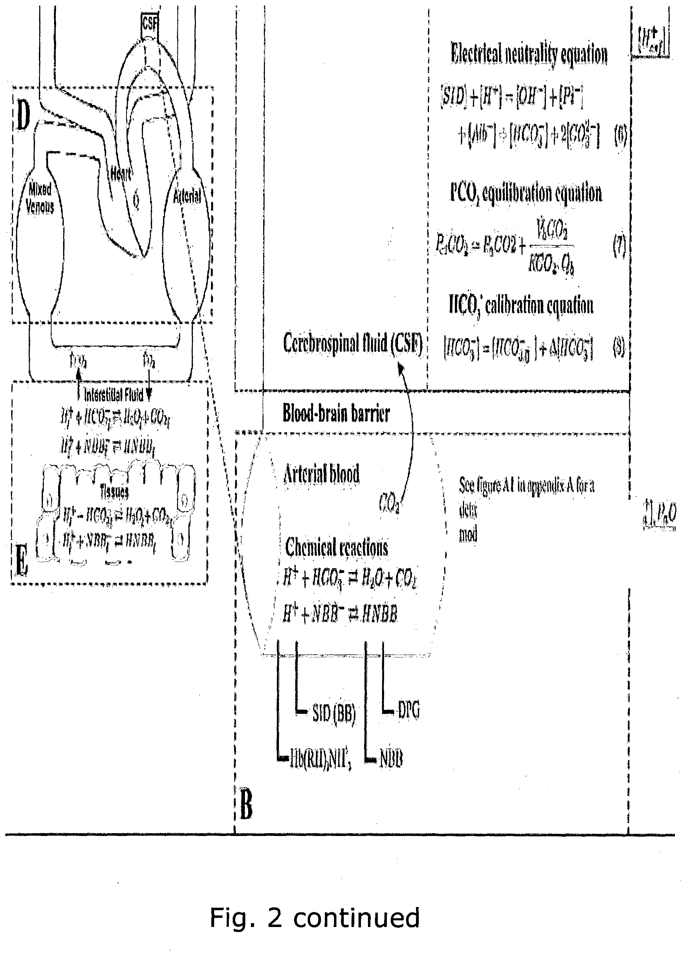

The number of organ systems and/or physiological systems that the computer system or the method of the invention is able to provide indication of may vary. Thus, in an embodiment the one or more physiological models being capable of modelling the patient over time by outputting a plurality of corresponding physiological parameters derivable from said first, said second and said third data describing the functioning of 2-10 organ systems and/or physiological systems simultaneous, such as 3-10, such as 5-10, such as 2-8, such as 4-8, or such as 4-6. The number organ systems and/or physiological systems that the computer system of the invention is able to provide indication of depends of the number of parameters included in the one or more physiological models or the number of physiological models the system comprises. Again, figure 2 shows examples of different model elements.

The invention is particularly, but not exclusively, advantageous for obtaining that monitoring and alarms may be based upon changes in the patient's physiological state rather than symptomatic changes described by direct measurements as hitherto. Furthermore, simulations performed by the models may be used to identify when the patient is poorly understood and to direct the clinician as to the appropriate further measurements.

In a second aspect, the present invention relates to a method for medical monitoring of a patient using one or more physiological models implemented on a computer system, the method comprising:
- providing first data (D1) indicative of characteristics in the inspired gas of the patient (1) to the computer system (2),
- providing second data (D2) indicative of characteristics in the respiratory feedback in expired gas of the patient (1) to the computer system (2), and
- providing third data (D3) indicative of the acid/base status and oxygenation level in the blood of the patient (1) to the computer system (2),
the computer system being arranged for:
i. performing a mathematical physiological modelling of the patient using said first, second and third data as a function of time, wherein the one or more physiological models being capable of modelling the patient over time by outputting a plurality of corresponding physiological parameters derivable from said first, said second and said third data describing the functioning of at least two organ systems and/or physiological systems simultaneous,
ii. performing a monitoring process wherein
   - if one, or more, of the physiological parameters have a deviation compared to a related threshold level, the computer system outputs:
      - an indication of the one or more organ systems, and/or one or more physiological systems causing said deviation; and
      - optionally, an alarm signal;
      and/or
   - if one or more of the data values have a deviation compared to a related threshold level, the computer system outputs:
      - an indication of one or more organ systems, and/or one or more physiological systems and/or one or more non-physiological systems causing said deviation; and
      - optionally, an alarm signal.

In a third aspect, the present invention relates to a computer program product being adapted to enable a computer system comprising at least one computer having data storage means in connection therewith to implement a method according to the second aspect.

The second and/or the third aspect may be adapted for implementation on an entity selected from the group consisting of a mechanical ventilation monitoring system, an intensive care monitor system, a portable computing device, a handheld mobile device, such as a tablet, and an electronic patient journal system, etc., as it will readily be the skilled person once the teaching and general principle of the invention is comprehended. In separate aspects, the invention may also relate to a mechanical ventilation monitoring system, an intensive care monitor system, a portable computing device or similar as such for implementing the invention.

The third aspect of the invention is particularly, but not exclusively, advantageous in that the present invention may be accomplished by a computer program product enabling a computer system to carry out the operations of the first aspect of the invention when down- or uploaded into the computer system. Such a computer program product may be provided on any kind of computer readable medium, or through a network.

The individual aspects of the present invention may each be combined with any of the other aspects. These and other aspects of the invention will be apparent from the following description with reference to the described embodiments.

### BRIEF DESCRIPTION OF THE FIGURES

The invention will now be described in more detail with regard to the accompanying figures. The figures show one way of implementing the present invention and is not to be construed as being limiting to other possible embodiments falling within the scope of the attached claim set.
Figure 1 is a schematic drawing of a method or a computer system according to the present invention.
Figure 2 illustrates a set of mathematical models of a decision support system (DSS) including the mathematical representation of a physiological model of respiratory control.
Figure 3 is a schematic drawing of a method according to the present invention.
Figure 4A illustrates how current monitoring and alarm approaches work, whereas figure 4B illustrates the situation where physiological parameters are determined simultaneously from the data values. Dot for SpO2 indicates that a threshold level has been breached and dot for lungs is indicative for a possible cause for the low SpO2 measured.
Figure 5A illustrates how current monitoring and alarm approaches work, whereas figure 5B illustrates the situation where physiological parameters are determined simultaneously from the data values. Dot for SpO2 indicates that a threshold level has been breached and dot for metabolism is indicative for a possible cause for the low SpO2 measured.
Figure 6A illustrates how current monitoring and alarm approaches work, whereas figure 6B illustrates the situation where physiological parameters are determined simultaneously from the data values. Dot for SpO2 in figure 6A indicates that a threshold level has been breached and the absence of a dot in figure 6B indicates that no physiological parameter is responsible for the low SpO2. Thus, a cause may be instrument failure.
Figure 7A illustrates how current monitoring and alarm approaches work, whereas figure 7B illustrates the situation where physiological parameters are determined simultaneously from the data values. Although no thresholds are breached in figure 7A, calculations of physiological parameters in figure 7B show that for both lungs and circulation thresholds levels have been breached.
Figure 8 shows a flow-chart of the method according to the present invention.

### DETAILED DESCRIPTION OF AN EMBODIMENT

This invention is a method/computer system for providing intelligent monitoring, and in doing so for providing intelligent alarms and directed measurements. Key to this invention is the application of physiological models to integrate the available measurements, and estimate parameters which describe the underlying causality. Simulations using these models can then be used to identify when patients underlying physiology changes, i.e. when measurements no longer match simulations, and as such to direct measurement strategy.

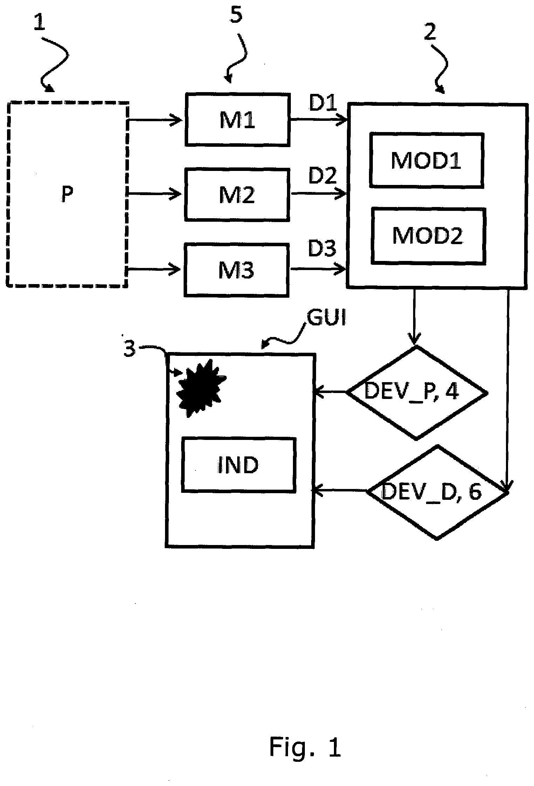

Figure 1 is a schematic drawing of a specific embodiment of the method or a computer system of the invention. The method or computer system is for providing assistance to a clinician by providing indications of reasons for an alarm in relation to patient values being outside a threshold range of an associated patient 1, P. The method comprises that a computer system 2 receives input data D1, D2, D3 from measuring means M1, M2, M3, respectively. The computer system is adapted to control whether the input data are positioned within a threshold range or outside said range for the specific value. If one or more of the values lie outside (or deviates significantly, DEV_D) the threshold range the system can set of an alarm 3 while also providing an indication IND of the reason for the data value lying outside the threshold range. Indication is calculated based on physiological models MOD1, MOD2 e.g. as displayed in figure 2 using the one or more data values in each model. In this way the clinician receives input to which measures must be taken to solve the problem. The system may also give indication to which further measurements/analysis may be performed on the patient to give better indication of the underlying problem.

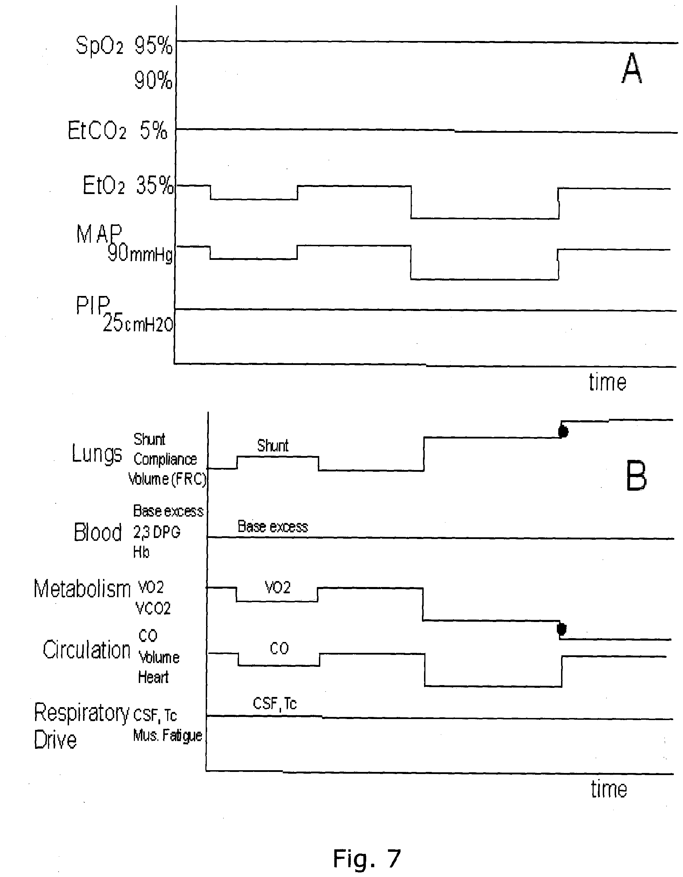

The situation may also arise where the input data values D1, D2, D3 do not lie outside any threshold ranges, but that after the input values have been used in one or more physiologic models one (or more) of the output physiological parameters lies outside (or deviates significantly, DEV_P) their specific threshold range. In this situation the system may also set of an alarm 3, e.g. a visible alarm, an audio alarm, and/or a tactile alarm, etc., and providing an indication IND of an underlying problem not detectable by only monitoring the data values. This situation is displayed in figure 7, where two changes in physiological parameters results in that no changes in data values can be observed. In this situation the method/ computer system is also adapted for providing the clinician with further advice on which further tests could be performed on the patient to getter further information which can improve the indication provided.

Figure 2 illustrates an example of a mathematical model (or several independent models) which might be used in the integration of current signals for provision of intelligent monitoring. The model includes components describing the lungs (A), the oxygenation and acid-base chemistry of the blood (B), the acid-base of cerebral spinal fluid (C), circulation (D), tissue metabolism and buffering (E) and respiratory drive (F). Further models which may be implemented include models of pulmonary mechanics or renal function. As such this model in figure 2 will be used in the following text to exemplify the method of the invention.

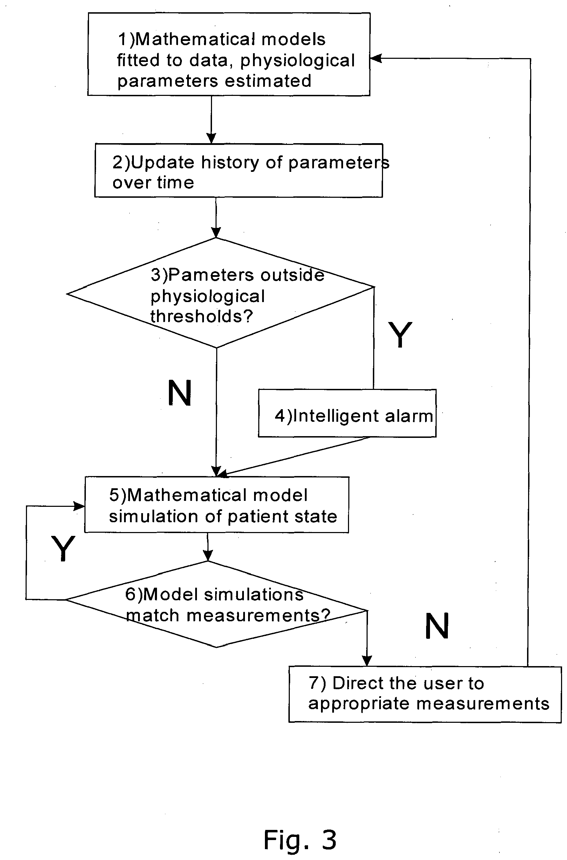

An embodiment of the method of the invention is illustrated in figure 3 and described in the following text. Physiological mathematical models, such as that illustrated in figure 2, often have specific variables known as parameters. Typical to these parameters is that they are often not measured directly, but estimated by fitting the model to measured data (step 1, figure 2). Parameter values provide a deep picture of the patients underlying physiology and are usually fixed, normally from estimated values, when performing simulations

For the model presented in figure 2 these include parameters describing the lungs (A), e.g. pulmonary shunt (A); the blood (B), e.g. base excess; CSF (C), e.g. CSF bicarbonate level; circulation (D), e.g. cardiac output; tissue metabolism (E), e.g. oxygen consumption or carbon dioxide production; or respiratory drive (F), e.g. central chemoreceptor drive threshold.

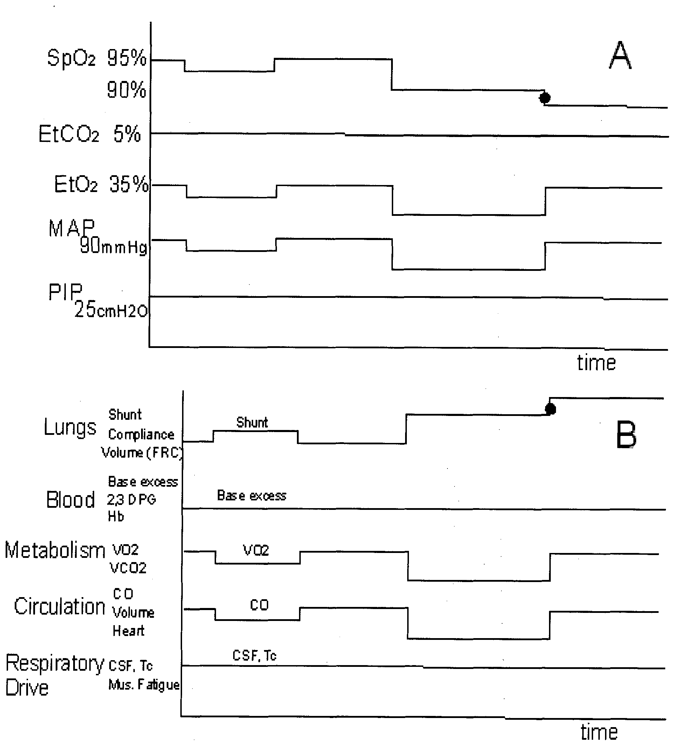

For a patient presenting at the ICU it is possible to measure continuously respiratory gasses (oxygen and carbon dioxide), respiratory flows and pressures, arterial oxygenation, blood pressures, cardiac output and tissue production of CO2 and consumption of O2. In addition, it is usual to measure arterial blood acid-base and oxygenation status periodically. These measurements can be used to identify the parameters of the models using model fitting techniques. By doing this periodically over time (step 2, figure 3) the monitoring strategy can be shifted from one of looking at the individual measurement signals, to changes in the underlying physiological systems. Figure 4 gives an example of this approach. Figure 4A illustrates how current monitoring and alarm approaches work. Each signal is displayed continuously and alarms given when a single parameter is outside a range of values considered appropriate (threshold levels) or if there is sensor disconnection. In this case an example is given where oxygen saturation, measured continuously as SpO2 falls below 90 % and an alarm threshold (dot) is passed. The signals represented in Figure 4A may be examples of first data D1 (e.g. PIP), second data D2 (e.g. EtCO2 and EtO2), and third data D3 (e.g. SpO2) according to the present invention. Blood pressure, MAP, may be an example of fifth data D5 according to the invention.

In contrast, figure 4B illustrates the situation where physiological parameters are monitored using the models of figure 2 according to the present invention. Parameters are displayed according to the organ system they represent. When any single system or organ function changes intelligent alarms can then be based upon a deeper understanding (steps 3,4 figure 3).

Thus, in figure 4 it is possible to provide an indication to the clinician stating that the fall in SpO2 may be due to increased shunt.

When models have been fitted to clinical data they can then be used to perform simulations (step 5, figure 3). This process can be used to perform directed measurements, the principle of which is as follows. By fixing model parameters the models can be used to simulate all variables. Those measured continuously can then be compared with model simulations. These may be for example those illustrated in figure 4A including arterial oxygen saturation measured with pulse oximetry, expiratory gas concentrations of oxygen and carbon dioxide, arterial blood pressure, or positive inspiratory pressure in the lungs or other respiratory measurements such as flow, frequency or minute ventilation. Differences between model simulations and measurements, are then an indication that patient physiology has changed and new measurements are required (step 6, figure 3). Thus, it is possible to use the model to identify the need for further measurements.

The next step is in directing the clinician to which measurements (step 7, figure 3). For example, an increase in expired CO₂ can be due to an increase in metabolism or a reduction in alveolar ventilation. Measurement of VCO2 will explain the metabolic component, through the model. A reduction in alveolar ventilation can be explained by a reduced respiratory drive or a change in acid-base status. Measurement of acid-base status requires arterial blood sampling so that the clinician could be directed toward this measurement. If the expiratory CO₂ levels do not match the arterial blood gas then this suggests a change in pulmonary gas exchange which would direct the clinician to measurements aiding in understanding pulmonary function. A different example of directed measurement would be a decrease in expiratory O₂. This can be due to increase in oxygen consumption or a change in pulmonary gas exchange. Measurement of oxygen consumption (VO2) would enable distinction between these two and measurement of pulse oximetry SpO₂ would indicate whether expiratory O2 levels input in the model simulated arterial oxygen levels. If not then the user can be directed to measure arterial blood oxygen levels should with a blood sample to confirm that these match SpO₂, if not then a measure of pulmonary gas exchange is necessary. This directed measurement is only possible through combination of several models, and model simulation to identify when measurements do not match simulations and as such what further measurements are required to understand the patient. New measurements would then require model re-fitting and restart the cycle shown in figure 4.

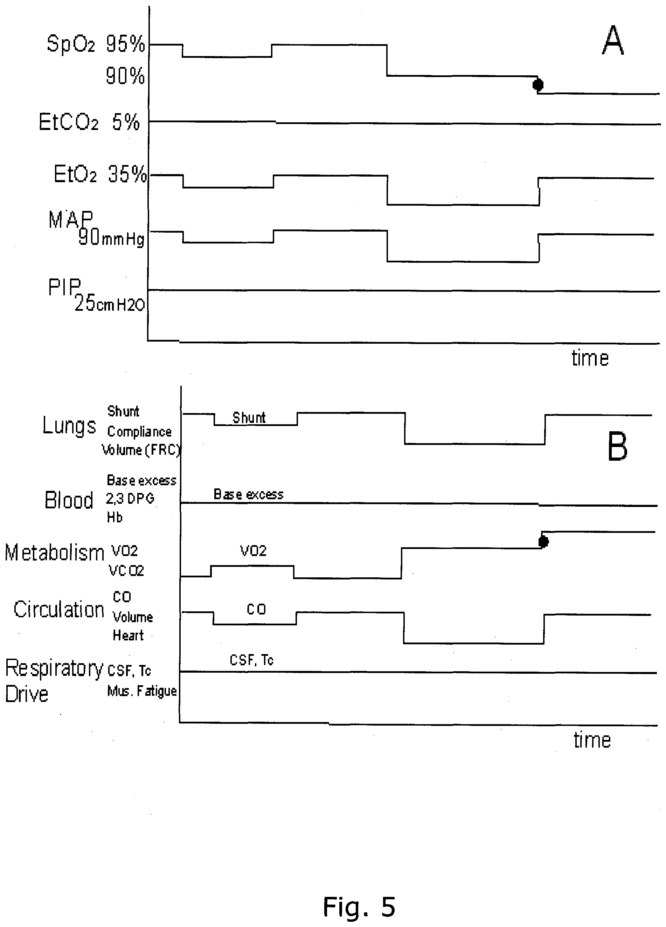

Figure 5 is an example wherein the same change in SpO2 has taken place as shown in figure 4, but where the cause for the drop in SpO2 is different; in this case an increase in metabolism.

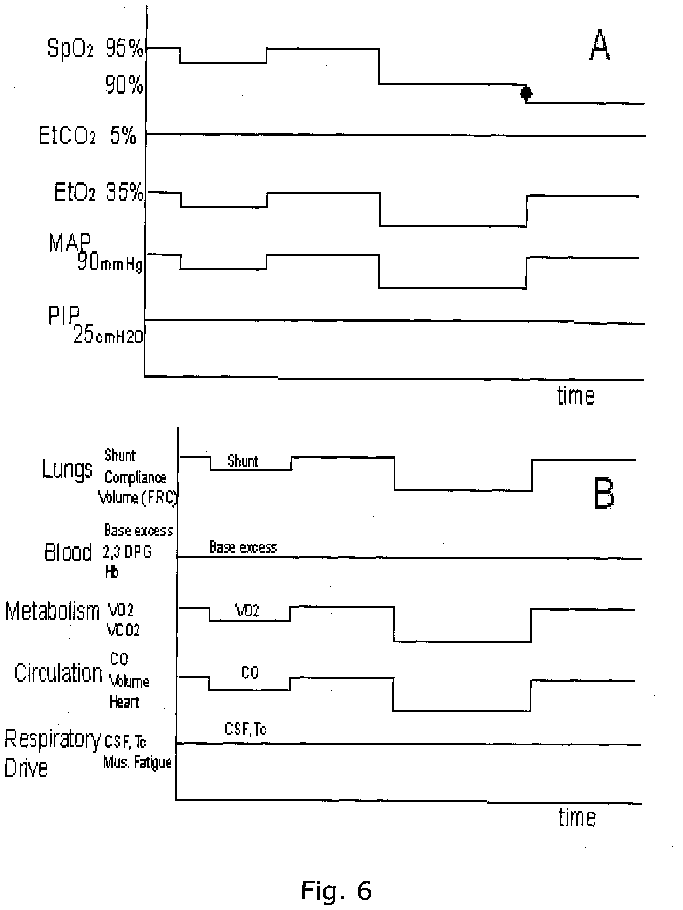

Figure 6 illustrates the situation where there can be change in patient data without apparent physiological change. SpO2 appears to reduce but without an increasing physiological problem. This can, for example be due to sensor miss-placement or failure.

Figure 7 illustrates the situation where there is no change in data but with two changes in physiological effect which can cancel each other out. An increasing pulmonary shunt in the lungs would reduce oxygen levels in the blood. However, if present with either a decreased use of oxygen in the tissue (i.e. reduction in VO2) or increased inspiratory oxygen, then no change would be seen in the oxygenation of the blood. Thus, if only standard monitoring of direct data input was used, the clinician would get no warning from the monitoring system that the patient health was worsening. By employing the method of the invention the clinician would get a warning that both lungs and metabolism were getting outside preferred threshold levels/ranges.

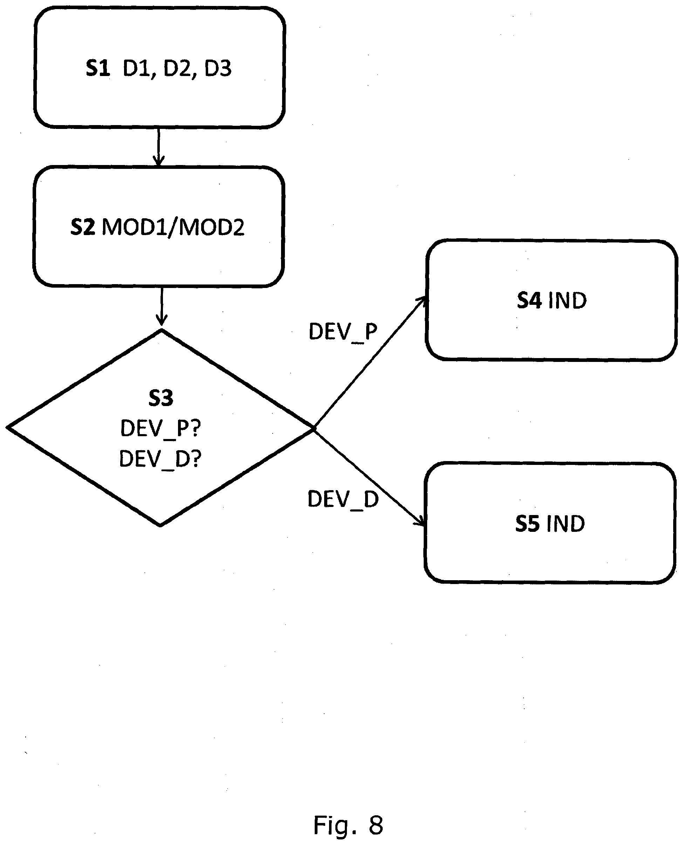

Figure 8 is a flow-chart of a method according to the present invention for medical monitoring of a patient P using one or more physiological models, MOD1 and/or MOD2, implemented on a computer system 2, the method comprising:
**S1a** providing first data D1 indicative of characteristics in the inspired gas of the patient 1 to the computer system 2,
**S1b** providing second data D2 indicative of characteristics in the respiratory feedback in expired gas of the patient 1 to the computer system 2, and
**S1c** providing third data D3 indicative of the acid/base status and oxygenation level in the blood of the patient 1 to the computer system 2,
the computer system being arranged for:
**S2** performing a mathematical physiological modelling of the patient using said first (D1), second (D2) and third (D3) data as a function of time, wherein the one or more physiological models (MOD1, MOD2) being capable of modelling the patient over time by outputting a plurality of corresponding physiological parameters derivable from said first, said second and said third data describing the functioning of at least two organ systems and/or physiological systems simultaneous,
**S3** performing a monitoring process wherein
   - **S4** if one, or more, of the physiological parameters have a deviation DEV_P compared to a related threshold level, the computer system outputs:
      - an indication IND of the one or more organ systems, and/or one or more physiological systems causing said deviation; and
      - optionally, an alarm signal 3;
      and/or
   - **S5** if one or more of the data values, D1, D2, and D3, have a deviation DEV_D, 6 compared to a related threshold level, the computer system outputs:
      - an indication IND of one or more organ systems, and/or one or more physiological systems and/or one or more non-physiological systems causing said deviation; and
      - optionally, an alarm signal 3.

### Glossary

- FiO₂: Fraction of oxygen in inspired gas.
- PiO₂: Pressure of oxygen in inspired gas.
- FiCO₂: Fraction of carbon dioxide in inspired gas.
- PiCO₂: Pressure of carbon dioxide in inspired gas.
- SaO₂: Oxygen saturation of arterial blood, measured from a blood sample.
- CaO₂: Oxygen concentration in arterial blood.
- PaO₂: Pressure of oxygen in arterial blood, measured from a blood sample.
- SpO₂: Oxygen saturation of arterial blood, measured transcutaneously.
- EtO2 / FE'O₂: Fraction of oxygen in expired gas at the end of expiration.
- FEO₂: Fraction of oxygen in the mixed expired gas.
- PE'O₂: Pressure of oxygen in expired gas at the end of expiration.
- PAO₂: Pressure of oxygen in mixed alveolar gas.
- PEO₂: Pressure of oxygen in the mixed expired gas.
- EtCO2 /FE'CO₂: Fraction of carbon dioxide in expired gas at the end of expiration.
- FECO₂: Fraction of carbon dioxide in the mixed expired gas.
- PE'CO₂: Pressure of carbon dioxide in expired gas at the end of expiration.
- PACO₂: Pressure of carbon dioxide in expired gas at the end of expiration.
- PECO₂: Pressure of oxygen in the mixed expired gas.
- PaCO₂: Carbon dioxide partial pressure in arterial blood, measured from a blood sample.
- CaCO₂: Carbon dioxide concentration in arterial blood.

- VO₂: Oxygen consumption, i.e. the litres of oxygen consumed by the tissues per minute.
- VCO₂: Carbon dioxide production, i.e. the litres of carbon dioxide produced by the tissues per minute.

- V: Ventilation.
- Vt: Tidal volume, i.e. volume of gas breathed per breath.
- f: Respiratory frequency, i.e. number of breaths per minute.
- Vd: Dead space i.e. the volume of the lung not involved in exchanging gases with the blood.
- Vmin: minute ventilation
- Valv: Alveolar ventilation
- Volume (FRC): Lung volume (functional residual capacity)
- fA2: fraction of ventilation to one of the ventilated compartments

- Q̇: Perfusion
- fs/ shunt: fraction of blood circulation not involved in gas exchange.
- f2: fraction of non-shunt blood circulation to one of the ventilated compartments
- pHa: Arterial blood pH
- H⁺: hydrogen ion concentration
- BE: Base excess
- HCO₃⁻: Bicarbonate concentration
- Alb: Albumin concentration
- NBB: Non-bicarbonate buffer concentration
- DPG: 2,3 Diphosphoglycerate concentration
- SID: Strong ion difference

- MAP: Mean arterial blood pressure
- PIP: Positive inspiratory pressure

- Tc, Sc, Tp Sp, A, Po: Model constants and parameters describing respiratory drive

- Kw', Alb⁻_{fix} Kc, K₃: Model constants and parameters describing the buffering properties of CSF (Cerebral spinal fluid)

- Dw, Dp, Dc: Respiratory drive: wakefulness (w); peripheral (p); and central (c)

- i: subscript for interstitial fluid
- a: subscript for arterial blood
- v: subscript for venous blood
- c: subscript for capillary blood

### References

1. Arnstein F. (1997) Catalogue of human error. Br J Anaesth. 79:645-656
2. Rees SE and Andreassen S. Mathematical models of oxygen and carbon dioxide storage and transport: the acid base chemistry of blood (2005). Crit Rev Biomed Eng. 33:209-264.

The invention can be implemented by means of hardware, software, firmware or any combination of these. The invention or some of the features thereof can also be implemented as software running on one or more data processors and/or digital signal processors.

The individual elements of an embodiment of the invention may be physically, functionally and logically implemented in any suitable way such as in a single unit, in a plurality of units or as part of separate functional units. The invention may be implemented in a single unit, or be both physically and functionally distributed between different units and processors.

Although the present invention has been described in connection with the specified embodiments, it should not be construed as being in any way limited to the presented examples. The scope of the present invention is to be interpreted in the light of the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. A computer system (2) for medical monitoring of a patient (P) using one or more physiological models (MOD1, MOD2) implemented on the computer system, the system being arranged for:
- receiving first data (D1) indicative of characteristics in the inspired gas of the patient (1),
- receiving second data (D2) indicative of characteristics in the respiratory feedback in expired gas of the patient (1), and
- receiving third data (D3) indicative of the acid/base status and oxygenation level in the blood of the patient (1),
the computer system being arranged for:
i. performing a mathematical physiological modelling of the patient using said first (D1), second (D2) and third (D3) data as a function of time, wherein the one or more physiological models (MOD1, MOD2) is/are capable of modelling the patient over time by outputting a plurality of corresponding physiological parameters derivable from said first, said second and said third data describing the functioning of at least two organ systems and/or physiological systems simultaneously,
ii. performing a monitoring process wherein
- if one, or more, of the physiological parameters describing the functioning of at least two organ systems and/or physiological systems have a deviation (DEV_P) compared to a related threshold level, the computer system outputs:
• an indication (IND) of the one or more organ systems, and/or one or more physiological systems causing said deviation; and
• optionally, an alarm signal (3),
thereby indicating an underlying cause for a change in the first (D1), second (D2) and third (D3) data or an underlying physiological change of the patient.

2. The computer system according to claim 1, wherein the indication of one or more organ systems, one or more physiological systems and/or non-physiological systems causing said deviation is based on
▪ current values of other physiological parameters and/or current data values; and/or
▪ previous values of the deviating physiological parameter and/or previous values of the data values.

3. The computer system according to any of the preceding claims, wherein the computer system is further arranged for
- providing fourth data (D4) indicative of characteristics of the blood circulation, such as cardiac output; and/or
- providing fifth data (D5) indicative of characteristics of heart function, such as blood pressure; and/or
- providing sixth data (D6) indicative of characteristics of kidney function, such as renal perfusion or bicarbonate reabsorption.

4. The computer system according to any of the preceding claims, wherein the one or more organ systems are chosen from the group consisting of lungs, blood, heart, kidneys, and tissues.

5. The computer system according to any of the preceding claims, wherein the one or more physiological systems are chosen from the group consisting of metabolism, circulation, renal function and respiration.

6. The computer system according to claim 1, wherein said modelling comprises a tuning process of one, or more, physiological parameters at regularly time intervals, preferably based on current and/or previous values of data (D1, D2, D3).

7. The computer system according to any of the preceding claims, wherein the computer system is arranged for, optionally when no physiological parameters have a deviation (DEV) compared to a related physiological threshold:
- performing a simulation of the patient state by keeping constant any physiological parameters from the modelling and simulating the time development of the patient state.

8. The computer system according to claim 7, wherein said simulation of the patient state over time is applied by comparing one, or more, physiological parameters and/or simulated physiological values corresponding to obtainable data with data (D1, D2, D3) to assess if the said simulation can adequately describe the patient state now, or at a later time.

9. The computer system according to claim 8, wherein if said simulation cannot describe the patient state now or at a later time, the computer system is arranged for indicating one or more possible causes therefore.

10. The computer system according to any of the preceding claims, wherein the computer system being arranged for indicating one or more further patient measurements which can be performed for obtaining further data values (D1, D2, D3) which may improve the said modelling, the said simulation and/or the said indications (IND) of possible deviations.

11. The computer system according to any of the preceding claims, wherein (ii) performing of a monitoring process wherein one, or more, of the physiological parameters have a deviation (DEV_P) compared to a related threshold level, there are no data values (D1, D2, D3) outside a threshold range (DEV_D).

12. The computer system according to any of the preceding claims, wherein the one or more physiological models (MOD1, MOD2) being capable of modelling the patient over time by outputting a plurality of corresponding physiological parameters derivable from said first, said second and said third data describing the functioning of 2-10 organ systems and/or physiological systems simultaneous, such as 3-10, such as 5-10, such as 2-8, such as 4-8, or such as 4-6.

13. A method for medical monitoring of a patient (P) using one or more physiological models (MOD1, MOD2) implemented on a computer system, the method comprising:
- providing first data (D1) indicative of characteristics in the inspired gas of the patient (1) to the computer system (2),
- providing second data (D2) indicative of characteristics in the respiratory feedback in expired gas of the patient (1) to the computer system (2), and
- providing third data (D3) indicative of the acid/base status and oxygenation level in the blood of the patient (1) to the computer system (2),
the computer system being arranged for:
i. performing a mathematical physiological modelling of the patient using said first (D1), second (D2) and third (D3) data as a function of time, wherein the one or more physiological models (MOD1, MOD2) is/are capable of modelling the patient over time by outputting a plurality of corresponding physiological parameters derivable from said first, said second and said third data describing the functioning of at least two organ systems and/or physiological systems simultaneously,
ii. performing a monitoring process wherein
- if one, or more, of the physiological parameters describing the functioning of at least two organ systems and/or physiological systems have a deviation (DEV_P) compared to a related threshold level, the computer system outputs:
• an indication (IND) of the one or more organ systems, and/or one or more physiological systems causing said deviation; and
• optionally, an alarm signal (3),
thereby indicating an underlying cause for a change in the first (D1), second (D2) and third (D3) data or an underlying physiological change of the patient.

14. A computer program product being adapted to enable a computer system comprising at least one computer having data storage means in connection therewith to implement a method according to claim 13.

15. The computer program product according to claim 14, being adapted for implementation on an entity selected from the group consisting of a mechanical ventilation monitoring system, an intensive care monitor system, a portable computing device, a handheld mobile device, such as a tablet, and an electronic patient journal system.

## Patentansprüche

1. Computersystem (2) zum medizinischen Überwachen eines Patienten (P) unter Verwendung von einem oder mehreren physiologischen Modellen (MOD1, MOD2), die im Computersystem umgesetzt sind, wobei das System für Folgendes vorgesehen ist:
- Empfangen von ersten Daten (D1), die Eigenschaften des eingeatmeten Gases des Patienten (1) anzeigen,
- Empfangen von zweiten Daten (D2), die Eigenschaften der Atmungsrückkopplung des ausgeatmeten Gases des Patienten (1) anzeigen, und
- Empfangen von dritten Daten (D3), die den Säure-Basen-Status und die Sauerstoffsättigung im Blut des Patienten (1) anzeigen,
wobei das Computersystem für Folgendes vorgesehen ist:
i. Durchführen einer mathematischen physiologischen Modellierung des Patienten unter Verwendung der ersten (D1), zweiten (D2) und dritten (D3) Daten in Abhängigkeit von der Zeit, wobei das eine oder die mehreren physiologischen Modelle (MOD1, MOD2) in der Lage ist/sind, den Patienten im Laufe der Zeit durch das Ausgeben einer Vielzahl von entsprechenden physiologischen Parametern zu modellieren, die aus den ersten, den zweiten und den dritten Daten ableitbar sind, die das Funktionieren von mindestens zwei Organsystemen und/oder physiologischen Systemen gleichzeitig beschreiben,
ii. Durchführen eines Überwachungsprozesses, wobei
- das Computersystem Folgendes ausgibt, wenn einer oder mehrere der physiologischen Parameter, die das Funktionieren von mindestens zwei Organsystemen und/oder physiologischen Systemen beschreiben, eine Abweichung (DEV_P) im Vergleich zu einem zugehörigen Schwellenwertpegel aufweisen:
• eine Anzeige (IND) des einen oder der mehreren Organsysteme und/oder des einen oder der mehreren physiologischen Systeme, welche die Abweichung verursachen; und
• optional, ein Alarmsignal (3),
wodurch eine zugrunde liegende Ursache für eine Änderung in den ersten (D1), zweiten (D2) und dritten (D3) Daten oder eine zugrunde liegende physiologische Änderung des Patienten angezeigt wird.

2. Computersystem nach Anspruch 1, wobei die Anzeige eines oder mehrerer Organsysteme, eines oder mehrerer physiologischer Systeme und/oder nichtphysiologischer Systeme, welche die Abweichung verursachen, auf Folgendem basiert:
▪ aktuellen Werten anderer physiologischer Parameter und/oder aktuellen Datenwerten; und/oder
▪ vorherigen Werten der abweichenden physiologischen Parameter und/oder vorherigen Werten der Datenwerte.

3. Computersystem nach einem der vorhergehenden Ansprüche, wobei das Computersystem ferner für Folgendes vorgesehen ist:
- Bereitstellen vierter Daten (D4), die Eigenschaften des Blutkreislaufs anzeigen, wie das Herzminutenvolumen; und/oder
- Bereitstellen fünfter Daten (D5), die Eigenschaften der Herzfunktion anzeigen, wie den Blutdruck; und/oder
- Bereitstellen sechster Daten (D6), die Eigenschaften der Nierenfunktion anzeigen, wie die Nierenperfusion oder die Bicarbonatreabsorption.

4. Computersystem nach einem der vorhergehenden Ansprüche, wobei das eine oder die mehreren Organsysteme aus der Gruppe ausgewählt sind, die aus Lungen, Blut, Herz, Nieren und Geweben besteht.

5. Computersystem nach einem der vorhergehenden Ansprüche, wobei das eine oder die mehreren physiologischen Systeme aus der Gruppe ausgewählt sind, die aus Stoffwechsel, Kreislauf, Nierenfunktion und Atmung besteht.

6. Computersystem nach Anspruch 1, wobei das Modellieren einen Abstimmprozess von einem oder mehreren physiologischen Parametern in regelmäßigen Zeitabständen umfasst, vorzugsweise auf Grundlage aktueller und/oder vorheriger Werte von Daten (D1, D2, D3).

7. Computersystem nach einem der vorhergehenden Ansprüche, wobei das Computersystem für Folgendes vorgesehen ist, wenn optional keine physiologischen Parameter eine Abweichung (DEV) im Vergleich zu einem zugehörigen physiologischen Schwellenwert aufweisen:
- Durchführen einer Simulation des Patientenzustands durch das Konstanthalten beliebiger physiologischer Parameter vom Modellieren und Simulieren des Zeitverlaufs des Patientenzustands.

8. Computersystem nach Anspruch 7, wobei die Simulation des Patientenzustands im Laufe der Zeit dadurch angewendet wird, dass einer oder mehrere physiologische Parameter und/oder simulierte physiologische Werte, die erhaltbaren Daten entsprechen, mit Daten (D1, D2, D3) verglichen werden, um zu beurteilen, ob die Simulation den Patientenzustand jetzt oder zu einem späteren Zeitpunkt angemessen beschreiben kann.

9. Computersystem nach Anspruch 8, wobei das Computersystem, wenn die Simulation den Patientenzustand jetzt oder zu einem späteren Zeitpunkt nicht beschreiben kann, für das Anzeigen eines oder mehrerer möglicher Ursachen dafür vorgesehen ist.

10. Computersystem nach einem der vorhergehenden Ansprüche, wobei das Computersystem zum Anzeigen von einer oder mehreren weiteren Patientenmessungen vorgesehen ist, die zum Erhalten weiterer Datenwerte (D1, D2, D3) durchgeführt werden können, die das Modellieren, die Simulation und/oder die Anzeigen (IND) möglicher Abweichungen möglicherweise verbessern.

11. Computersystem nach einem der vorhergehenden Ansprüche, wobei (ii) das Durchführen eines Überwachungsprozesses, wobei einer oder mehrere der physiologischen Parameter eine Abweichung (DEV_P) im Vergleich zu einem zugehörigen Schwellenwertpegel aufweisen, keine Datenwerte (D1, D2, D3) außerhalb eines Schwellenwertbereichs (DEV_D) ergeben.

12. Computersystem nach einem der vorhergehenden Ansprüche, wobei das eine oder die mehreren physiologischen Modelle (MOD1, MOD2) in der Lage sind, den Patienten im Laufe der Zeit durch das Ausgeben einer Vielzahl von entsprechenden physiologischen Parametern zu modellieren, die von den ersten, den zweiten und den dritten Daten ableitbar sind, die das Funktionieren von 2-10 Organsystemen und/oder physiologischen Systemen gleichzeitig beschreiben, wie 3-10, wie 5-10, wie 2-8, wie 4-8 oder wie 4-6.

13. Verfahren zum medizinischen Überwachen eines Patienten (P) unter Verwendung von einem oder mehreren physiologischen Modellen (MOD1, MOD2), die auf einem Computersystem umgesetzt sind, wobei das Verfahren Folgendes umfasst:
- Bereitstellen für das Computersystem (2) von ersten Daten (D1), die Eigenschaften des eingeatmeten Gases des Patienten (1) anzeigen,
- Bereitstellen für das Computersystem (2) von zweiten Daten (D2), die Eigenschaften der Atmungsrückkopplung des ausgeatmeten Gases des Patienten (1) anzeigen, und
- Bereitstellen für das Computersystem (2) von dritten Daten (D3), die den Säure-Basen-Status und die Sauerstoffsättigung im Blut des Patienten (1) anzeigen,
wobei das Computersystem für Folgendes vorgesehen ist:
i. Durchführen einer mathematischen physiologischen Modellierung des Patienten unter Verwendung der ersten (D1), zweiten (D2) und dritten (D3) Daten in Abhängigkeit von der Zeit, wobei das eine oder die mehreren physiologischen Modelle (MOD1, MOD2) in der Lage ist/sind, den Patienten im Laufe der Zeit durch das Ausgeben einer Vielzahl von entsprechenden physiologischen Parametern zu modellieren, die aus den ersten, den zweiten und den dritten Daten ableitbar sind, die das Funktionieren von mindestens zwei Organsystemen und/oder physiologischen Systemen gleichzeitig beschreiben,
ii. Durchführen eines Überwachungsprozesses, wobei
- das Computersystem Folgendes ausgibt, wenn einer oder mehrere der physiologischen Parameter, die das Funktionieren von mindestens zwei Organsystemen und/oder physiologischen Systemen beschreiben, eine Abweichung (DEV_P) im Vergleich zu einem zugehörigen Schwellenwertpegel aufweisen:
• eine Anzeige (IND) des einen oder der mehreren Organsysteme und/oder des einen oder der mehreren physiologischen Systeme, welche die Abweichung verursachen; und
• optional, ein Alarmsignal (3),
wodurch eine zugrunde liegende Ursache für eine Änderung in den ersten (D1), zweiten (D2) und dritten (D3) Daten oder eine zugrunde liegende physiologische Änderung des Patienten angezeigt wird.

14. Computerprogrammprodukt, das dazu angepasst ist, einem Computersystem, das mindestens einen Computer mit Datenspeichermitteln in Verbindung damit umfasst, zu ermöglichen, ein Verfahren nach Anspruch 13 umzusetzen.

15. Computerprogrammprodukt nach Anspruch 14, das zur Umsetzung auf einem Element angepasst ist, das aus der Gruppe ausgewählt ist, die aus einem mechanischen Beatmungsüberwachungssystem, einer Intensivpflegeüberwachungseinheit, einer tragbaren Rechenvorrichtung, einer tragbaren mobilen Vorrichtung, wie ein Tablett, und einem elektronischen Patientenaktensystem besteht.

## Revendications

1. Système informatique (2) de surveillance médicale d'un patient (P) utilisant un ou plusieurs modèles physiologiques (MOD1, MOD2) mis en oeuvre sur le système informatique, le système étant conçu pour :
- recevoir des premières données (D1) indiquant des caractéristiques dans le gaz inspiré du patient (1),
- recevoir des deuxièmes données (D2) indiquant des caractéristiques dans le retour respiratoire dans le gaz expiré du patient (1), et
- recevoir des troisièmes données (D3) indiquant l'état acide/base et le niveau d'oxygénation dans le sang du patient (1),
le système informatique étant conçu pour :
i. effectuer une modélisation physiologique mathématique du patient à l'aide desdites premières (D1), deuxièmes (D2) et troisièmes (D3) données en fonction du temps, dans lequel les un ou plusieurs modèles physiologiques (MOD1, MOD2) est/sont capable(s) de modéliser le patient au fil du temps en émettant une pluralité de paramètres physiologiques correspondants pouvant être dérivés desdites premières, desdites deuxièmes et desdites troisièmes données décrivant le fonctionnement d'au moins deux systèmes d'organes et/ou systèmes physiologiques simultanément,
ii. effectuer un processus de surveillance dans lequel
- si un ou plusieurs des paramètres physiologiques décrivant le fonctionnement d'au moins deux systèmes d'organes et/ou systèmes physiologiques présentent un écart (DEV_P) par rapport à un niveau de seuil associé, le système informatique émet :
• une indication (IND) des un ou plusieurs systèmes d'organes, et/ou des un ou plusieurs systèmes physiologiques provoquant ledit écart ; et
• éventuellement, un signal d'alarme (3),
indiquant ainsi une cause sous-jacente d'un changement dans les premières (D1), deuxièmes (D2) et troisièmes (D3) données ou d'un changement physiologique sous-jacent du patient.

2. Système informatique selon la revendication 1, dans lequel l'indication d'un ou de plusieurs systèmes d'organes, d'un ou de plusieurs systèmes physiologiques et/ou systèmes non physiologiques provoquant ledit écart est basée sur
▪ des valeurs actuelles d'autres paramètres physiologiques et/ou valeurs de données actuelles ; et/ou
▪ des valeurs précédentes du paramètre physiologique et/ou des valeurs précédentes d'écart des valeurs de données.

3. Système informatique selon l'une quelconque des revendications précédentes, dans lequel le système informatique est en outre conçu pour
- fournir des quatrièmes données (D4) indiquant des caractéristiques de la circulation sanguine, telles que le débit cardiaque ; et/ou
- fournir des cinquièmes données (D5) indiquant des caractéristiques de la fonction cardiaque, telles que la pression artérielle ; et/ou
- fournir des sixièmes données (D6) indiquant des caractéristiques de la fonction rénale, telles que la perfusion rénale ou la réabsorption de bicarbonate.

4. Système informatique selon l'une quelconque des revendications précédentes, dans lequel les un ou plusieurs systèmes d'organes sont choisis dans le groupe constitué des poumons, du sang, du coeur, des reins et des tissus.

5. Système informatique selon l'une quelconque des revendications précédentes, dans lequel les un ou plusieurs systèmes physiologiques sont choisis dans le groupe constitué du métabolisme, de la circulation, de la fonction rénale et de la respiration.

6. Système informatique selon la revendication 1, dans lequel ladite modélisation comprend un processus de réglage d'un ou de plusieurs paramètres physiologiques à des intervalles de temps réguliers, de préférence sur la base de valeurs actuelles et/ou antérieures de données (D1, D2, D3).

7. Système informatique selon l'une quelconque des revendications précédentes, dans lequel le système informatique est conçu pour, éventuellement, lorsqu'aucun paramètre physiologique ne présente d'écart (DEV) par rapport à un seuil physiologique associé :
- effectuer une simulation de l'état du patient en maintenant tous les paramètres physiologiques constants à partir de la modélisation et de la simulation du développement temporel de l'état du patient.

8. Système informatique selon la revendication 7, dans lequel ladite simulation de l'état du patient au fil du temps est appliquée par la comparaison d'un(e) ou de plusieurs paramètres physiologiques et/ou valeurs physiologiques simulées correspondant à des données pouvant être obtenues avec des données (D1, D2, D3) pour évaluer si ladite simulation peut décrire de manière adéquate l'état du patient à présent, ou à un moment ultérieur.

9. Système informatique selon la revendication 8, dans lequel, si ladite simulation ne peut pas décrire l'état du patient à présent ou à un moment ultérieur, le système informatique est conçu pour indiquer une ou plusieurs causes possibles en découlant.

10. Système informatique selon l'une quelconque des revendications précédentes, dans lequel le système informatique est conçu pour indiquer une ou plusieurs autres mesures de patient qui peuvent être effectuées pour obtenir d'autres valeurs de données (D1, D2, D3) qui peuvent améliorer ladite modélisation, ladite simulation et/ou lesdites indications (IND) d'écarts possibles.

11. Système informatique selon l'une quelconque des revendications précédentes, dans lequel (ii), lors de la mise en oeuvre d'un processus de surveillance dans lequel un ou plusieurs des paramètres physiologiques présentent un écart (DEV_P) par rapport à un niveau de seuil associé, il n'y a pas de valeurs de données (D1, D2, D3) en dehors d'une plage de seuil (DEV_D).

12. Système informatique selon l'une quelconque des revendications précédentes, dans lequel les un ou plusieurs modèles physiologiques (MOD1, MOD2) sont capables de modéliser le patient au fil du temps en émettant une pluralité de paramètres physiologiques correspondants pouvant être dérivés desdites premières, desdites deuxièmes et desdites troisièmes données décrivant le fonctionnement simultané de 2 à 10 systèmes d'organes et/ou systèmes physiologiques, tels que de 3 à 10, tels que de 5 à 10, tels que de 2 à 8, tels que de 4 à 8, ou tels que de 4 à 6.

13. Procédé de surveillance médicale d'un patient (P) utilisant un ou plusieurs modèles physiologiques (MOD1, MOD2) mis en oeuvre sur un système informatique, le procédé comprenant :
- la fourniture de premières données (D1) indiquant des caractéristiques dans le gaz inspiré du patient (1) au système informatique (2),
- la fourniture de deuxièmes données (D2) indiquant des caractéristiques dans le retour respiratoire dans le gaz expiré du patient (1) au système informatique (2), et
- la fourniture de troisièmes données (D3) indiquant l'état acide/base et le niveau d'oxygénation dans le sang du patient (1) au système informatique (2),
le système informatique étant conçu pour :
i. effectuer une modélisation physiologique mathématique du patient à l'aide desdites premières (D1), deuxièmes (D2) et troisièmes (D3) données en fonction du temps, dans lequel les un ou plusieurs modèles physiologiques (MOD1, MOD2) est/sont capable(s) de modéliser le patient au fil du temps en émettant une pluralité de paramètres physiologiques correspondants pouvant être dérivés desdites premières, desdites deuxièmes et desdites troisièmes données décrivant le fonctionnement d'au moins deux systèmes d'organes et/ou systèmes physiologiques simultanément,
ii. effectuer un processus de surveillance dans lequel
- si un ou plusieurs des paramètres physiologiques décrivant le fonctionnement d'au moins deux systèmes d'organes et/ou systèmes physiologiques présentent un écart (DEV_P) par rapport à un niveau de seuil associé, le système informatique émet :
• une indication (IND) des un ou plusieurs systèmes d'organes, et/ou des un ou plusieurs systèmes physiologiques provoquant ledit écart ; et
• éventuellement, un signal d'alarme (3),
indiquant ainsi une cause sous-jacente d'un changement dans les premières (D1), deuxièmes (D2) et troisièmes (D3) données ou d'un changement physiologique sous-jacent du patient.

14. Produit de programme informatique conçu pour permettre à un système informatique comprenant au moins un ordinateur ayant un moyen de stockage de données en connexion avec celui-ci afin de mettre en oeuvre un procédé selon la revendication 13.

15. Produit de programme informatique selon la revendication 14, conçu pour la mise en oeuvre, sur une entité choisie dans le groupe constitué d'un système de surveillance de ventilation mécanique, d'un système de surveillance de soins intensifs, d'un dispositif informatique portable, d'un dispositif mobile portatif, tel qu'une tablette, et d'un système de journal électronique des patients.
